**Europäisches Patentamt**

**European Patent Office**

**Office européen des brevets**

(19)

(11) Veröffentlichungsnummer: **0 201 023**
**B1**

# EUROPÄISCHE PATENTSCHRIFT

(12)

(45) Veröffentlichungstag der Patentschrift:
18.01.89

(51) Int. Cl.⁴: **A 61 B 17/58**

(21) Anmeldenummer: **86105850.1**

(22) Anmeldetag: **28.04.86**

(54) Osteosyntheseplatte für die Druckstabilisierung.

(30) Priorität: **06.05.85 DE 8513286 U**

(43) Veröffentlichungstag der Anmeldung:
**12.11.86 Patentblatt 86/46**

(45) Bekanntmachung des Hinweises auf die Patenterteilung:
**18.01.89 Patentblatt 89/3**

(84) Benannte Vertragsstaaten:
**AT BE CH DE FR GB IT LI NL SE**

(56) Entgegenhaltungen:
**DE-U- 8 309 326**
**US-A- 3 528 085**
**US-A- 3 547 114**
**US-A- 3 604 414**

(73) Patentinhaber: **Wolter, Dietmar Prof. Dr.,
Lohmühlenstrasse 5, D-2000 Hamburg 1 (DE)**

(72) Erfinder: **Wolter, Dietmar Prof. Dr., Lohmühlenstrasse 5,
D-2000 Hamburg 1 (DE)**

(74) Vertreter: **Glawe, Delfs, Moll & Partner Patentanwälte,
Postfach 26 01 62 Liebherrstrasse 20,
D-8000 München 26 (DE)**

## Beschreibung

Die Erfindung bezieht sich auf eine Osteosyntheseplatte für die Druckstabilisierung von Knochenfragmenten.

In der Osteosynthese ist es bekannt, dass die Knochenheilung durch Stabilität und Druck im Knochenspalt gefördert wird. Es sind daher Spannvorrichtungen entwickelt worden, die in Verbindung mit Osteosyntheseplatten die Druckstabilisierung der Knochenfragmente ermöglichen. Dazu gehören Osteosyntheseplatten, deren Schraubenlöcher in der Art einer schiefen Ebene geneigte Lochwände, im folgenden als Rampen bezeichnet, aufweisen, so dass beim Einschrauben einer Knochenschraube der Schraubenkopf auf der Rampe gleitet und damit eine Verschiebung der Platte im Verhältnis zu dem die Knochenschraube aufnehmenden Knochenfragment bewirkt. Eine Osteosyntheseplatte dieser Gattung ist aus US-A-3 528 085 bekannt. Der grösstmögliche Kompressionsweg, der durch die Lochgeometrie und die Dicke der Platte bestimmt ist, ist dabei sehr kurz. Grössere Kompressionsstrecken lassen sich mit Hilfe einer besonderen Spannvorrichtung erreichen, die zusätzlich zur Osteosyntheseplatte angesetzt wird. Wenn diese Vorrichtung ausserhalb der Knochenplatte an einem Knochenfragment festgeschraubt wird, hat sie den Nachteil einer grösseren Wunde, grösseren operativen Aufwands und längerer Operationszeit. Wenn sie im Bereich der Osteosyntheseplatte in Verbindung mit einem in dieser vorgesehenen Langloch angebracht wird (DE-OS 3 134 120), kann je nach Art der Bruchform und Lage des Langlochs eine eingeschränkte Versorgungsmöglichkeit gegeben sein.

Der Erfindung liegt die Aufgabe zugrunde, eine Osteosyntheseplatte zu schaffen, die für die Druckstabilisierung die Einfachheit und bewährte Wirksamkeit der Rampenlöcher nutzt und dennoch eine grössere Kompressionsstrecke ermöglicht.

Die erfindungsgemässe Lösung besteht darin, dass mehrere demselben Knochenfragment zugeordnete Langlöcher mit jeweils mehreren Rampenlochabschnitten vorgesehen sind, wobei die Teilungsabstände der demselben Langloch angehörenden Rampenlochabschnitte nicht grösser sind als die Zahl der demselben Knochenfragment zugeordneten Langlöcher multipliziert mit der nutzbaren Rampenlänge. Um mit lediglich zwei Langlöchern auskommen zu können, sind die Teilungsabstände der Rampenlochabschnitte zweckmässigerweise geringer als die doppelte nutzbare Rampenlänge.

Die erfindungsgemässe Osteosyntheseplatte wird in dieser Weise genutzt, dass Knochenschrauben in den unterschiedlichen Langlöchern alternierend eingedreht und wieder gelöst werden. Nachdem eine erste Knochenschraube innerhalb eines ersten Langlochs in einem darin der Fraktur nahegelegenen Rampenlochabschnitt eingedreht worden ist, wodurch ein erster Kompressionsschritt durchgeführt wurde, wird eine zweite Knochenschraube in einem anderen Langloch so angesetzt, dass sie in den Anfang einer Rampe eingreift. Danach wird die erste Schraube gelöst und durch Eindrehen der zweiten Schraube ein zweiter Kompressionsschritt durchgeführt. Wenn die Teilungsabstände der Rampenlochabschnitte geringer sind als die doppelte nutzbare Rampenlänge, hat in diesem zweiten Kompressionsschritt die erste Schraube den Beginn eines weiteren Rampenlochabschnitts erreicht, wird mit diesem in Eingriff gebracht, worauf die zweite Schraube gelöst werden kann und die erste Schraube wieder eingedreht wird, was einen dritten Kompressionsschritt ergibt usw. Wenn drei Langlöcher vorhanden sind und die nutzbare Rampenlänge einem Drittel der Teilungsabstände entspricht, werden in entsprechender Weise drei Schrauben alternierend eingedreht und gelöst.

Auf diese Weise lassen sich beliebig lange Kompressionsstrecken überwinden. Dies ist insbesondere bei Osteotomien, beispielsweise im Bereich des Hüftgelenks, wichtig, weil es dabei notwendig werden kann, zur Kompression der Schnittflächen Wege von mehreren Millimetern bis in die Grössenordnung von 1 cm zu überwinden.

Selbstverständlich ist dieses Prinzip auch für die gegenüber der Kompression umgekehrte Bewegungsrichtung zum Zwecke der Dekompression einsetzbar. Diese Möglichkeit, bei der die Rampenanordnung in den den beiden Knochenfragmenten zugeordneten Plattenabschnitten umgekehrt ist, soll daher von dem Schutzrecht gleichfalls umfasst sein.

Die Erfindung wird im folgenden näher unter Bezugnahme auf die Zeichnung erläutert. Darin zeigen:

Fig. 1 eine schematische Draufsicht auf die erfindungsgemässe Platte und

Fig. 2 in vergrösserter, schematischer Darstellung das Funktionsprinzip.

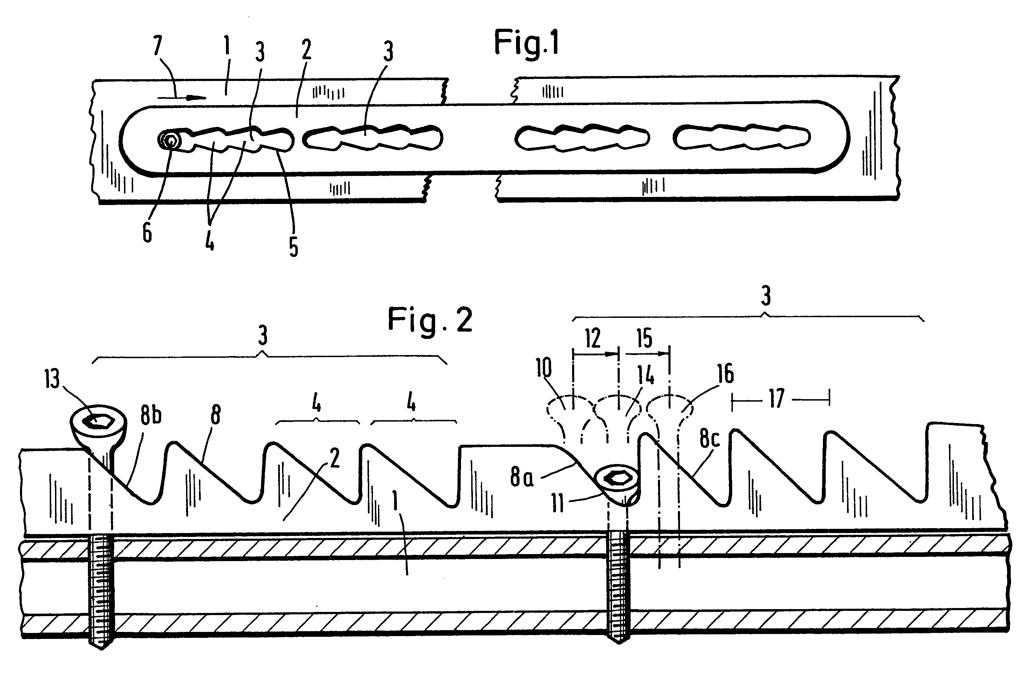

Die in Fig. 1 auf einem Knochen 1 mit einer Querfraktur liegende Osteosyntheseplatte 2 enthält in ihrer jedem der beiden Knochenfragmente zugeordneten Abschnitte zwei Langlöcher 3, die jeweils vier Lochabschnitte 4, die jeweils als ein Paar von einander gegenüberliegenden Begrenzungsbögen 5 erkennbar sind und jeweils mit einer Rampe ausgerüstet sind und deshalb hier als Rampenlochabschnitte bezeichnet werden, aufweisen. Die Rampen auf der linken Seite in Fig. 1 sind fallend nach rechts angeordnet, während die Rampen auf der rechten Seite fallend nach links angeordnet sind, so dass sich beim Eindrehen der bei 6 in Fig. 1 angeordneten Knochenschraube eine Bewegung des zugehörigen Knochenfragments in Pfeilrichtung 7, also in Kompressionsrichtung, ergibt. Einzelheiten dieser Funktion werden nun anhand von Fig. 2 dargestellt, die den in Fig. 1 linken Abschnitt der Osteosyntheseplatte — stark vergrössert gegenüber dem Knochen 1 — darstellt. Die Bereiche, in denen sich die Langlöcher 3 befinden, sind in Fig. 2 durch entsprechende Klammern angegeben. Darin sind die den jeweiligen Rampenlochabschnitten 4 zugeordneten Rampen 8 als schiefe Ebenen erkennbar.

Zunächst wird eine erste Knochenschraube in dem in Fig. 2 rechten Langloch in deren der Fraktur fernen Ende eingesetzt in Position 10. Beim Eindrehen gleitet sie mit ihrem Schraubenkopf die schiefe Ebene 8a hinab und nimmt dabei den darunter liegenden Knochen 1 mit, der dadurch um die Strecke 12 in Pfeilrichtung verschoben wird. Nun wird die zweite Knochenschraube über dem in der Zeichnung linken Langloch an dessen der Fraktur fernen Ende eingesetzt in Position 13 und eingedreht, bis sie an der zugehörigen schiefen Ebene 8b anschlägt und dadurch den Knochen 1 in der erreichten Position gegenüber der Osteosyntheseplatte sichert. Dann wird die erste Schraube gelockert bis zur Position 14, in der sie sich frei über der Platte gefindet. Durch das Eindrehen der zweiten Schraube verschiebt sich der Knochen um den Abschnitt 15 in Pfeilrichtung, wobei die erste Schraube aus der Position 14 in Position 16 wandert, in der sie sich über der Rampe 8c des nächsten Rampenlochabschnitts befindet. Sie kann nun mit dieser Rampe in Eingriff gebracht werden, worauf die zweite Schraube gelockert wird, usw. Durch das alternierende Eindrehen und Lösen der Schraube kommt es auf diese Weise zu einer beliebig langen Kompressionsstrecke.

In dem dargestellten Beispiel ist die nutzbare Rampenlänge jeweils etwa gleich den Abschnitten 12 bzw. 15 oder ein wenig grösser. Es handelt sich dabei um diejenige Strecke, um die der Knochen gegenüber der Osteosyntheseplatte verschoben wird, wenn eine Schraube sich vom äussersten einen Ende einer Rampe bis zum äussersten anderen Ende derselben Rampe bewegt. Der Teilungsabstand zwischen zwei Rampenlochabschnitten ist in Fig. 2 mit 17 bezeichnet.

### Patentansprüche

1. Osteosyntheseplatte für die Druckstabilisierung von Knochenfragmenten, dadurch gekennzeichnet, dass mehrere demselben Knochenfragment (1) zugeordnete Langlöcher (3) mit jeweils mehreren Rampenlochabschnitten (4) vorgesehen sind, wobei die Teilungsabstände (17) der demselben Langloch (3) angehörenden Rampenlochabschnitte (4) nicht grösser sind als die Zahl der demselben Knochenfragment zugeordneten Langlöcher multipliziert mit der nutzbaren Rampenlänge (12, 15).

2. Osteosyntheseplatte nach Anspruch 1, dadurch gekennzeichnet, dass die Teilungsabstände (17) geringer sind als die doppelte nutzbare Rampenlänge.

### Claims

1. An osteosynthetic plate for the compression stabilisation of bone fragments, characterised in that a plurality of oblong holes (3) associated with the same bone fragment (1) are each provided with a plurality of ramp hole portions (4), wherein the pitch spacing (17) of the ramp hole portions (4) belonging to one oblong hole (3) are not greater than the number of the oblong holes associated with the same bone fragment multiplied by the effective ramp length (12, 15).

2. An osteosynthetic plate according to Claim 1, characterised in that the pitch spacings (17) are smaller than double the effective ramp length.

### Revendications

1. Plaque d'ostéosynthèse pour la stabilisation de fragments osseux par pression, caractérisée en ce qu'il est prévu plusieurs trous oblongs (3) associés au même fragment osseux (1) et comportant chacun plusieurs segments de trou en rampe (4), les distances de partage (17) des segments de trou en rampe (4) appartenant au même trou oblong (3) n'étant pas plus grandes que le nombre des trous oblongs associés au même fragment osseux, multiplié par la longueur de rampe utilisable (12, 15).

2. Plaque d'ostéosynthèse selon la revendication 1, caractérisée en ce que les distances de partage (17) sont plus petites que le double de la longueur de rampe utilisable.

# Fig.1

# Fig. 2